(19) Europäisches Patentamt
European Patent Office
Office européen des brevets

(11) Veröffentlichungsnummer: **0 221 022 A2**

(12) **EUROPÄISCHE PATENTANMELDUNG**

(21) Anmeldenummer: **86810463.9**

(22) Anmeldetag: **20.10.86**

(51) Int. Cl.4: **C 07 C 79/36**, C 07 C 76/00

(30) Priorität: **24.10.85 CH 4580/85**

(43) Veröffentlichungstag der Anmeldung: **06.05.87 Patentblatt 87/19**

(84) Benannte Vertragsstaaten: **CH DE ES FR GB IT LI**

(71) Anmelder: **CIBA-GEIGY AG, Klybeckstrasse 141, CH-4002 Basel (CH)**

(72) Erfinder: **Comninellis, Christos, Dr., Route Les Esserts, CH-1030 Mex (CH)**
Erfinder: **Osterwalder, Robert Ulrich, Palmenstrasse 20, CH-4055 Basel (CH)**
Erfinder: **Plattner, Eric, Prof. Dr., Jurastrasse 18, CH-4411 Seltisberg (CH)**
Erfinder: **Seifert, Gottfried, Mühlenmattweg 20, CH-4312 Magden (CH)**

(54) **Verfahren zur Herstellung von 5-Nitro-1,4-naphthochinonen.**

(57) Beschrieben wird ein Verfahren zur Herstellung von 5-Nitro-1,4-naphthochinonen der Formel

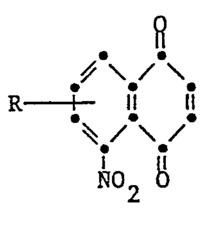

worin R Wasserstoff, $C_1$-$C_4$-Alkyl oder $C_1$-$C_4$-Alkoxy ist, durch Oxidation von 1-Nitronaphthalin oder entsprechend substituierter 1-Nitronaphthaline, das dadurch gekennzeichnet ist, dass man die Reaktion mit Mangan-III-sulfat als Oxidationsmittel durchführt.

Das verbrauchte Mangan-III-sulfat lässt sich mit guter Stromausbedeute elektrolytisch regenerieren. Das Verfahren ist umweltfreundlich, da das Oxidationsmittel und gegebenenfalls auch das Lösungsmittel wiederverwendet werden.

EP 0 221 022 A2

CIBA-GEIGY AG
Basel (Schweiz)

1-15544/=/CIT

# Verfahren zur Herstellung von 5-Nitro-1,4-naphthochinonen

Die Erfindung betrifft ein Verfahren zur Herstellung von 5-Nitro-1,4-naphthochinonen, durch Oxidation der entsprechenden 1-Nitronaphthaline mittels Mangan-III-sulfat.

5-Nitro-1,4-naphthochinon, im folgenden kurz 5-Nitronaphthochinon genannt, ist ein wichtiges Farbstoffzwischenprodukt, das man mit Butadien nach Diels-Alder zum 5-Nitro-1,4,11,12-tetrahydroanthrachinon umsetzt (siehe z.B. N.N. Vorozhtsov et al. Zhur. Vsesoyuz. Khim. Obshchestva im D.I. Mendeleeva 5, 474 (1960) - Chem. Abstract Vol. 55, 1547e), welches man anschliessend zum 1-Hydroxylaminoanthrachinon umlagert oder direkt in das 1-Aminoanthrachinon, der Ausgangsverbindung zahlreicher Dispersions- und Küpenfarbstoffe, überführt (siehe z.B. DE-A-24 50 883).

In der Literatur sind mehrere Verfahren zur Herstellung von 5-Nitronaphthochinon beschrieben. Nach den beiden wichtigsten Verfahrensvarianten geht man entweder aus von Naphthalin, das zunächst in 1-Stellung nitriert (Ullmann Bd. 17 (1979) Seite 392) und anschliessend oxidiert wird oder direkt vom 1,4-Naphthochinon, das man mit Nitriersäure in 5-Stellung nitriert (Ullmann, Bd. 17 (1979) Seite 121). Das im Vorliegenden beschriebene Verfahren betrifft die erste Variante und zwar die Oxidationsreaktion unter Verwendung von 1-Nitronaphthalin oder substituierten Nitronaphthalinen als Ausgangsmaterial.

Aus ökonomischen und ökologischen Gründen ist man heute mehr und mehr daran interessiert, Oxidationsmittel zu verwenden, die sich am Ende der Reaktion leicht aus dem Reaktionsgemisch zurückgewinnen und regenerieren lassen, um anschliessend wieder für eine weitere Oxidationsreaktion eingesetzt zu werden. So wird Naphthochinon beispielsweise durch Oxidation von Naphthalin mittels Cer-IV-sulfat erhalten, wobei man mit geschmolzenem Naphthalin arbeitet und das Cer-IV-sulfat aus dem während der Reaktion gebildeten Cer-III-sulfat mittels Elektrolyse oder Ozonolyse regeneriert (DE-A-32 20 305). Nach einem ähnlichen Verfahren gelingt auch die Herstellung von 5-Nitronaphthochinon aus 1-Nitronaphthalin (DE-A-23 01 803), wobei als Oxidationsmittel Ammoniumpersulfat verwendet wird in Kombination mit Cer-IV-sulfat und Silbernitrat. Durchgeführt wird das Verfahren in einem 2-Phasensystem aus wässriger und organischer Phase und das verbrauchte Persulfat wird durch Elektrolyse regeneriert.

Bei der Suche nach weiteren Oxidationsmitteln, die sich leicht elektrochemisch regenerieren lassen, wurde gefunden, dass 1-Nitronaphthalin auch mit Mangan-III-sulfat zum 5-Nitronaphthochinon oxidiert werden kann. Diese Methode ist hinsichtlich des Oxidationsmittels kostengünstig und das gebildete Mangan-II-sulfat lässt sich problemlos, mit guter Stromausbeute durch Elektrolyse wieder zum Mangan-III-sulfat reoxidieren und dann erneut verwenden. Auch erzielt man mit diesem Oxidationsmittel eine gute Raum-Zeit-Ausbeute, da relativ konzentriert gearbeitet werden kann.

Gegenstand der vorliegenden Erfindung ist somit ein Verfahren zur Herstellung von 5-Nitro-1,4-naphthochinonen der Formel

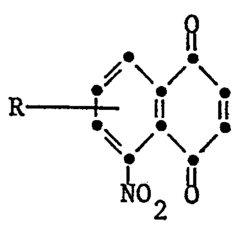

worin R Wasserstoff, ein Alkylrest mit 1 bis 4 C-Atomen oder ein Alkoxyrest mit 1 bis 4 C-Atomen ist, durch Oxidation von 1-Nitronaphthalin oder entsprechend substituierter 1-Nitronaphthaline, das dadurch gekennzeichnet ist, dass man die Reaktion mit Mangan-III-sulfat als Oxidationsmittel durchführt.

Die als Ausgangsmaterial verwendeten 1-Nitronaphthaline sind bekannt oder nach bekannten Methoden herstellbar. Das im vorliegenden beschriebene Verfahren dient in erster Linie zur Herstellung des unsubstituierten 5-Nitronaphthochinons ausgehend von 1-Nitronaphthalin.

Das 1-Nitronaphthalin oder substituierte 1-Nitronaphthaline werden zweckmässigerweise entweder direkt in geschmolzener Form mit einer schwefelsauren Mangan-III-sulfatlösung oder -suspension behandelt oder zuvor in einem mit Wasser nicht oder nur beschränkt mischbaren Lösungsmittel gelöst und diese Lösung wird dann mit der schwefelsauren Mangan-III-sulfatlösung bzw. -suspension intensiv vermischt. Als Lösungsmittel kommen natürlich nur solche in Frage, die unter den gewählten Reaktionsbedingungen inert sind, also weder durch das Mangan-III-salz oxidiert, noch durch die Schwefelsäure angegriffen werden. Man verwendet in erster Linie apolare aprotische Lösungsmittel, vor allem aromatische oder aliphatische Kohlenwasserstoffe, die bei Raumtemperatur flüssig sind, insbesondere Chlorbenzol, Dichlorbenzol oder Nitrobenzol. Ferner sind als Lösungsmittel geeignet chlorierte Biphenyle, 1,2,4-Trichlorbenzol oder Tetrachloräthylen; die Lösungsmittel können sowohl einzeln als auch im Gemisch eingesetzt werden, beispielsweise technische Dichlorbenzolgemische. Bei den aliphatischen Lösungsmitteln handelt es sich in erster Linie um unverzweigte und/oder verzweigte aliphatische Kohlenwasserstoffe, deren Siedebereich in einem Temperaturintervall von 130 bis 220°C liegt; geeignet sind z.B. Isoparaffingemische mit Siedebereichen von 155 bis 175°C, 170 bis 190°C bzw. 190 bis 210°C.

Auf 1 Teil Nitronaphthalin verwendet man zweckmässigerweise 0,5 bis 5 Teile, insbesondere 1 bis 2 Teile Lösungsmittel. Das Nitronaphthalin muss nicht vollständig gelöst sein, es kann auch als Emulsion oder im Lösungsmittel dispergiert vorliegen.

Das Mangan-III-sulfat wird in Form einer schwefelsauren Lösung oder Suspension eingesetzt. Ueblicherweise arbeitet man mit einer 30- bis 50%igen Suspension von Mangan-III-sulfat in Schwefelsäure einer Konzentration von ca. 30 bis 60%, insbesondere 40 bis 55%. Was das Verhältnis von Nitronaphthalin zu Mangan-III-sulfat anbetrifft, so verwendet man auf 1 Mol Nitronaphthalin zweckmässigerweise 1 bis zu 6 Mol, insbesondere 2 bis 5 Mol, Mangan-III-sulfat. Geringere Mengen als 1 Mol führen zu unrentabel kleinen Umsätzen, während eine Menge von mehr als 6 Mol Mangan-III-sulfat pro Mol Nitronaphthalin die Selektivität der Umsetzung verschlechtert. Verbessern lässt sich die Ausbeute durch einen Zusatz an Cersulfat, wobei bereits katalytische Mengen ausreichen. Es erweist sich als vorteilhaft, das Cersulfat $[Ce_2(SO_4)_3]$ in einer Konzentration von 0,1 bis 10 Mol%, insbesondere 1 bis 5 Mol% bezogen auf die Menge an Mangan-III-sulfat, einzusetzen.

Die elektrolytische Reoxidation des Mangan-II-sulfats kann in jeder beliebigen Elektrolysezelle durchgeführt werden. Als Kathode kommen die, für elektrochemische Reaktionen üblichen Werkstoffe, wie etwa Metalle, z.B. Blei, Metall-Legierungen, aktivierte Metalle, Metalloxid-Elektroden, Kohle-Elektroden, "Glassy-Carbon®"-Elektroden, in Frage; als Anode Platin, Blei, "Glassy-Carbon®" und $PbO_2$ auf Titan. Da die chemische Oxidation und die elektrochemische Reoxidation des verbrauchten Oxidationsmittels in getrennten Schritten nacheinander durchgeführt werden, kann in einer Elektrolysezelle ohne Diaphragma gearbeitet werden. Durchgeführt wird die Elektrolyse üblicherweise bei einer Spannung von 2 bis 5 Volt und einer Stromdichte von ca. 400 mA pro $cm^2$. Das Mangan-II-sulfat wird mit einem Umsatz von über 90% zu Mangan-III-sulfat reoxidiert.

Die chemische Oxidation des Nitronaphthalins zum Nitronaphthochinon wird allgemein bei einer Temperatur von 40 bis 90°C, insbesondere 55 bis 70°C durchgeführt, während die Elektrolyse üblicherweise bei einer Temperatur von 50 bis 100°C abläuft.

Die gemäss vorliegendem Verfahren erhaltenen 5-Nitronaphthochinone werden auf übliche Weise aus dem Reaktionsgemisch isoliert. Beim Arbeiten ohne Lösungsmittel wird das Produkt zusammen mit nicht umgesetztem Ausgangsmaterial aus dem schwefelsauren Reaktionsgemisch mit Hilfe eines organischen Lösungsmittels extrahiert. Zur Extraktion verwendet man vor allem Benzol oder Benzolderivate, wie z.B. Chlorbenzole, Nitrobenzol, Xylol oder insbesondere Toluol. Man verwendet zweckmässigerweise ein Lösungsmittel, in dem das als Ausgangsmaterial eingesetzte Nitronaphthalin auch in der Kälte gut löslich ist, aus dem das gebildete Nitronaphthochinon jedoch beim Abkühlen ausfällt und so durch einfache Kühlungskristallisation isoliert werden kann. Das ausgefallene Produkt wird abfiltriert und getrocknet. Man erhält so 5-Nitronaphthochinon bzw. die entsprechenden Alkyl- oder Alkoxyderivate mit einer Reinheit von 90 bis 95%. Das im Lösungsmittel verbliebene Nitronaphthalin kann zurückgeführt und in einem weiteren Oxidations-Zyklus als Ausgangsmaterial eingesetzt werden. Die anorganische Phase wird in eine Elektrolysezelle geleitet und das verbrauchte Mangan-III-sulfat elektrolytisch regeneriert.

Wird die erfindungsgemässe Reaktion in einem 2-Phasensystem durchgeführt, dann gestaltet sich die Aufarbeitung denkbar einfach. Nachdem die Reaktion beendet ist, trennt man die beiden Phasen voneinander. Aus der anorganischen Phase extrahiert man, zweckmässigerweise mit dem bereits in der Reaktion verwendeten Lösungsmittel, Reste an Produkt und Ausgangsmaterial und führt diese dann der vorstehend beschriebenen Elektrolyse zu, während man die organischen Phasen miteinander vereinigt und aus diesen nach bekannten Methoden, z.B. auch wieder durch Kühlungskristallisation, das gebildete Nitronaphthochinon isoliert. Das nichtumgesetzte Nitronaphthalin wird auch hier wieder als Ausgangsmaterial eingesetzt. Das Lösungsmittel wird ebenfalls zurückgewonnen und im nächsten Ansatz wiederverwendet.

Es fallen somit und das ist der grosse Vorteil des erfindungsgemässen Verfahrens, unabhängig davon ob in Gegenwart oder ohne Lösungsmittel gearbeitet wird, keine umweltbelastenden Rückstände an.

Die folgenden Beispiele dienen der Veranschaulichung der Erfindung; Teile bedeuten Gewichtsteile und Prozente Gewichtsprozente.

Beispiel 1

173 Teile 1-Nitronaphthalin werden in einem beheizbaren und mit einem Rührer und einer Pt/AgCl-Elektrode ausgerüsteten Reaktor vorgelegt und durch Erhitzen auf 60°C geschmolzen. Zu der Schmelze lässt man innerhalb von ca. 30 Minuten eine Mischung aus 2200 Teilen einer ca. 36%igen schwefelsauren Mangan-III-sulfatsuspension und 470 Teilen Wasser zulaufen. Das molare Verhältnis von 1-Nitronaphthalin zu Mangan-III-sulfat beträgt 1:2. Ferner enthält die Mangansulfatsuspension noch 0,9 Mol% Cer-III-sulfat, bezogen auf die Menge an Mangan-III-sulfat. Die Wassermenge wird so bemessen, dass die Schwefelsäurekonzentration am Ende der Reaktion 42 bis 43% beträgt. Das Reaktionsgemisch wird bei einer Temperatur von 60°C gerührt, bis das Mangan-III-sulfat verbraucht ist und sich vollständig zum Mangan-II-sulfat umgesetzt hat, was durch eine Potentialmessung leicht festgestellt werden kann. Aus dem Reaktionsgemisch werden anschliessend das gebildete 5-Nitro-1,4-naphthochinon und nichtumgesetztes 1-Nitronaphthalin mit insgesamt 225 Teilen Toluol extrahiert. Die produkthaltige Toluol-Phase wird mit insgesamt 1500 Teilen Wasser gewaschen und dann auf 0° bis 5°C abgekühlt, wobei das 5-Nitro-1,4-naphthochinon auskristallisiert. Das Produkt wird abfiltriert,mit 50 Teilen Toluol von 0°C gewaschen und anschliessend im Vakuum getrocknet. Die Ausbeute an 5-Nitro-1,4-naphthochinon beträgt 50 bis 60% der Theorie, bezogen auf umgesetztes 1-Nitronaphthalin. Das Produkt hat eine Reinheit von 90 bis 95%.

Die Mutterlauge wird bei 70°C unter Vakuum eingedampft und der Rückstand unter Berücksichtigung des 1-Nitronaphthalin-Anteils beim Folgeansatz wieder eingesetzt.

Beispiel 2

In einen beheizbaren Doppelmantelreaktor mit Rührer werden 100 Teile 1-Nitronaphthalin in 150 Teilen Chlorbenzol vorgelegt und auf 70°C erhitzt. Anschliessend werden 1526 Teile schwefelsaure Mangan-III-sulfat-Suspension eingetragen. Die Schwefelsäurekonzentration der Mangansulfatsuspension beträgt 45% und der Gehalt an Mangan-III-sulfat ca. 36%. Ferner enthält die Mangansulfatsuspension ca. 5 Mol% Cersulfat, bezogen auf die Menge an Mangan-III-sulfat. Man arbeitet mit etwas weniger als der stöchiometrischen Menge an Mangan-III-sulfat; auf 1 Mol Nitronaphthalin kommen ca. 2,4 Mol $Mn_2(SO_4)_3$. Die Mangan-III-sulfat-Suspension wird portionsweise während 1 bis 2 Stunden langsam zugegeben. Man lässt während 40 Minuten bei einer Temperatur von 70°C reagieren und stellt dann den Rührer ab. Nach Trennung der Phasen erhält man 238 Teile organische Phase mit einem Gehalt an Nitronaphthochinon von 18% und an Nitronaphthalin von 20%. Die schwefelsaure Phase schüttelt man mit 50 Teilen Chlorbenzol aus und erhält so weitere 50,5 Teile einer organischen Phase mit einem Gehalt von jeweils 4% an Nitronaphthochinon bzw. Nitronaphthalin. Insgesamt erhält man 38,3 Teile 5-Nitro-1,4-naphthochinon, das entspricht einer Ausbeute von 77% bezogen auf umgesetztes Nitronaphthalin. 49,6 Teile Nitronaphthalin werden zurückgewonnen. Das Chlorbenzol wird zu 98% zurückgewonnen, sowohl das Nitronaphthalin, wie auch das Chlorbenzol werden in einem folgenden Ansatz wiederverwendet.

Beispiel 3

In einem beheizbaren Doppelmantelreaktor mit Ankerrührer wird ein Gemisch aus 70 Teilen 1-Nitronaphthalin und 100 Teilen Nitrobenzol vorgelegt und auf 60°C erhitzt. Dann werden 994 Teile schwefelsaure Mangan-III-sulfat-Suspension eingetragen. Die Schwefelsäurekonzentration der Mangansulfatsuspension beträgt 52,6% und der Gehalt an Mangen-III-sulfat ca. 35%, das entspricht einer Menge von ca. 2 Mol Mangan-III-sulfat pro Mol 1-Nitronaphthalin. Ferner enthält die Mangansulfatsuspension noch ca. 1 Mol% Cersulfat, bezogen auf die Menge an Mangan-III-sulfat. Die Zugabe der Mangansulfatsuspension erfolgt portionsweise innerhalb von 1 1/2 bis 2 Stunden, anschliessend lässt man 105 Minuten reagieren. Dann wird der Rührer abgeschaltet und man wartet 15 Minuten bis vollständige Phasentrennung eingetreten ist. Die beiden Phasen werden voneinander getrennt und man erhält 165 Teile organische Phase mit einem Gehalt an Nitronaphthochinon von 20% und einem Gehalt an Nitronaphthalin von 22%. Man lässt die organische Phase auf etwa 20°C abkühlen und filtriert das ausgefallene Produkt ab, das man anschliessend bei 70°C trocknet. Man erhält 13 Teile Produkt, das zu 85% aus 5-Nitronaphthochinon besteht. Schmelzpunkt: 138-140°C. Durch Extrahieren der anorganischen Phase mit Nitrobenzol wird weiteres Produkt isoliert; insgesamt erhält man 28,1 Teile 5-Nitronaphthochinon, das entspricht einer Ausbeute von 73% bezogen auf umgesetztes 1-Nitronaphthalin.

Beispiel 4

Herstellung der schwefelsauren Mangan-III-sulfat-Suspension durch elektrochemische Oxidation von Mangan-II-sulfat.

In einem mit einem Rührer ausgerüsteten Reaktor werden 8 Teile Cer-III-sulfat, 500 Teile Mangan-II-sulfat·Monohydrat, 450 Teile Wasser und 650 Teile 95%ige Schwefelsäure miteinander vermischt. Man erhält so 1608 Teile Elektrolyt mit einer Schwefelsäurekonzentration von 55,5% und einem Gehalt an Mangan-II von 1,84 Mol/kg Elektrolyt. Der so vorbereitete Elektrolyt wird in eine mit Bleielektroden ausgerüstete Elektrolysezelle gefüllt und unter folgenden Bedingungen das Mangan-IIzum Mangan-III-sulfat oxidiert: Temperatur 85°C, Stromdichte 400 $mA/cm^2$, Spannung 3,1 bis 3,3 Volt. Nach 3 Stunden Elektrolyse, was einem Stromdurchgang von 120 A·h entspricht, sind 95% des Mangan-II-sulfats zum Mangan-III-sulfat oxidiert, das entspricht einer Stromausbeute von 69%.

Auf die gleiche Weise wird das am Ende der chemischen Oxidationsreaktion, in deren Verlauf das 1-Nitronaphthalin in das 5-Nitro-1,4-naphthochinon übergeführt wird, entstehende Mangan-II-sulfat wieder zum Mangan-III-sulfat reoxidiert und kann so in einem nachfolgenden Ansatz erneut als Oxidationsmittel verwendet werden.

Patentansprüche

1. Verfahren zur Herstellung von 5-Nitro-1,4-naphthochinon der Formel

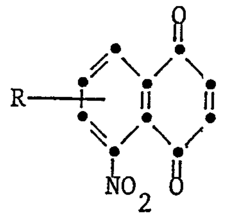

worin R Wasserstoff, $C_1$-$C_4$-Alkyl oder $C_1$-$C_4$-Alkoxy ist, durch Oxidation von 1-Nitronaphthalin oder entsprechend substituierter 1-Nitronaphthaline, dadurch gekennzeichnet, dass man die Reaktion mit Mangan-III-sulfat als Oxidationsmittel durchführt.

2. Verfahren gemäss Anspruch 1, dadurch gekennzeichnet, dass man in einem 2-Phasensystem aus geschmolzenem Nitronaphthalin und schwefelsaurer Mangan-III-sulfatlösung oder -suspension arbeitet.

3. Verfahren gemäss Anspruch 1, dadurch gekennzeichnet, dass man das 1-Nitronaphthalin gelöst in einem apolaren aprotischen Lösungsmittel einsetzt.

4. Verfahren gemäss Anspruch 3, dadurch gekennzeichnet, dass man als Lösungsmittel einen bei Raumtemperatur flüssigen aromatischen oder aliphatischen Kohlenwasserstoff verwendet.

5. Verfahren gemüss Anspruch 4, dadurch gekennzeichnet, dass man als Lösungsmittel Chlorbenzol, Dichlorbenzol oder Nitrobenzol verwendet.

6. Verfahren gemäss den Ansprüchen 1 bis 5, dadurch gekennzeichnet, dass man auf 1 Mol Nitronaphthalin 1 bis 6 Mol, insbesondere 2 bis 5 Mol Mangan-III-sulfat verwendet.

7. Verfahren gemäss Anspruch 1, dadurch gekennzeichnet, dass man als Oxidationsmittel Mangan-III-sulfat im Gemisch mit Cersulfat verwendet.

8. Verfahren gemäss Anspruch 7, dadurch gekennzeichnet, dass man 0,1 bis 10 Mol% Cersulfat, bezogen auf Mangan-III-sulfat, verwendet.

9. Verfahren gemäss Anspruch 8, dadurch gekennzeichnet, dass man 1 bis 5 Mol% Cersulfat, bezogen auf Mangan-III-sulfat, verwendet.

10. Verfahren gemäss Anspruch 1, dadurch gekennzeichnet, dass das während der Reaktion entstehende Mangan-II-sulfat mittels Elektrolyse zu Mangan-III-sulfat reoxidiert und erneut als Oxidationsmittel verwendet wird.

11. Die nach dem Verfahren gemäss Anspruch 1 erhaltenen 5-Nitro-1,4-naphthochinone.

FO 7.1/DOE/sch*